# EUROPEAN PATENT APPLICATION

(11) **EP 2 191 819 A1**
(43) Date of publication of application: **02.06.2010**
(21) Application number: 09252698.7
(22) Date of filing: 30.11.2009
(51) Int. Cl.: A61K 8/97, A61Q 5/06, A61Q 19/04

(54) **Methods for darkening the skin and/or hair**

(30) Priority: 01.12.2008 US 325434
(71) Applicant: Johnson & Johnson Consumer Companies, Inc., Skillman, NJ 08558 (US)
(72) Inventor: Chen, Nannan, Princeton, New Jersey 08540 (US); Lin, Connie B., Belle Mead, New Jersey 08502 (US); Zhang, Li, Princeton, New Jersey 08540 (US)
(74) Representative: Kirsch, Susan Edith

(57) **Abstract**

The present invention relates to a method of darkening the skin or hair by topically applying thereto a skin-darkening effective amount of a lotus leaf extract.

## Description

The present invention relates to the use of lotus leaf extract in darkening the skin and/or hair.

### BACKGROUND OF THE INVENTION

Many individuals desire the darkening of skin color. Most people obtain darker skin through exposure to UV light (e.g., sun-tanning or UV lamps). UV exposure, however, results in accelerated skin aging and increased incidence of skin cancer. The ability to generate a tanned appearance without incurring photo-damage, thus, is important. Accordingly, alternative methods for "sunless tanning" have evolved.

Products containing dihydroxy acetone (DHA) are well known as sunless tanners. These products, however, produce color that may be too orange, uneven, or unnatural to the user. DHA-containing products are continuously being improved (see, e.g., US Patent Application Publication 2007/0003496) to provide faster and more uniform color development, and long-lasting tan. Improved sunless tanning applications, including the use of a pressurised DHA spray apparatus, or skin warming procedures (see, e.g., WO 2007/057686), or the addition of skin bonding polymers (WO 2006/118638 and 2006/1109) recite further improvement in the even distribution and the long lasting effect of DHA, however the color characteristics of DHA- induced tanning remain unnatural. Products containing beta-carotene, cantaxanthin and lycopene have also been used to darken the skin. These products, however, have no effect on melanogenesis and usually result in unnatural and uneven skin color. Melanotan and MelanX are synthetic hormone drugs that mimic the action of melanocyte-stimulating hormone (MSH) and are used to darken the skin only when administered by injection. These agents are not available orally or topically. Psoralens work by making the skin hypersensitive to the sun and therefore accelerate melanin production. Psoralens require UV exposure, which must be carefully regulated to minimize UV-induced risks such as skin aging and cancer. Psoralens, in conjunction with medical grade UV lamps, are an accepted treatment for people afflicted with vitiligo and psoriasis, but are not recommended for patients with fair skin. Moreover, these alternatives do not provide the preferred "sunless tanning", and many individuals desire to use cosmetic, naturally derived skin care products to obtain darker skin color. Thus, a cosmetic product is desired that is safe, effective and inexpensive, which provides an even, natural skin color. Moreover, it would also be desirable to employ natural active ingredients.

Applicants have now discovered that extract of lotus leaf provides skin darkening benefits when used topically. Lotus extracts, particularly from the seed, are said to provide anti-aging benefits when used topically (US Patent No. 6,468,564) or orally (US Patent No. 6,602,526). Lotus extracts are also said to be useful in weight loss compositions (US Patent No. 7,074,440).

### SUMMARY OF THE INVENTION

The present invention provides a method of darkening the skin or hair comprising topically applying thereto a composition comprising a skin-darkening effective amount of a lotus leaf extract.

The present invention also provides a product comprising a) a composition comprising a skin-darkening effective amount of a lotus leaf extract; and b) instructions directing a user to apply said composition topically to the user's skin to darken the skin or even the skin tone or to the user's hair to darken the hair.

The present invention also provides a composition for use in darkening the composition skin or hair, or evening skin tone, the composition comprising a skin-darkening effective amount of a lotus leaf extract, wherein the composition is topically applied to the skin or hair.

The present invention also provides for a use of a composition comprising a lotus leaf extract for darkening the skin or hair or evening the skin tone, wherein the composition is topically applied to the skin or hair.

### DETAILED DESCRIPTION OF THE INVENTION

As used herein, "darkening" is darkening the appearance of human skin or hair, including, but not limited to, darkening human skin to either achieve a "sun tan" effect or to cover the light areas of the skin (e.g., as a result of a scar or a disease or a therapy) or darkening natural hair color or restoring discolored hair due to aging (e.g., gray or white hair) or external aggressions (e.g., excess exposure to sun or chlorine).

As used herein, a "product" is a product in finished packaged form. In one embodiment, the package is a container such as a plastic, metal or glass tube or jar containing the composition. The product may further contain additional packaging such as a plastic or cardboard box for storing such container. In one embodiment, the product contains instructions directing the user to apply the composition to the skin or hair to darken the skin (e.g., to tan the skin), even skin tone (e.g., to darken light areas of the skin or to treat or prevent mottled hyperpigmentation), or darken the hair (e.g., to darken light brown, blonde, gray or white hairs). Such instructions may be printed on the container, label insert, or on any additional packaging.

As used herein, "topically applying" means directly laying on or spreading on outer skin, the scalp, or hair, e.g., by use of the hands or an applicator such as a wipe, roller, or spray.

As used herein, "cosmetically acceptable" means that the ingredients the term describes are suitable for use in contact with tissues (e.g., the skin or hair) without undue toxicity, incompatibility, instability, irritation, allergic response, or the like.

As used herein, a "skin darkening effective amount" means an amount sufficient to induce a darkening of human skin or hair. This amount will vary with the area being treated, the age and skin or hair type of the end user, the duration and nature of the treatment, the specific composition employed, the carrier utilized, and like factors.

### Lotus Leaf Extract

According to the invention, a skin-darkening effective amount of a lotus leaf extract is topically applied to the skin or hair. The extract employed is from the leaf of the lotus plant (e.g. *Nelumbo nucifera*). Applicants have found that the leaf, in particular, provides darkening benefits that other parts of the lotus plant do not provide.

The lotus leaf extract is a blend of compounds isolated from the leaves of the lotus plant, e.g. from *Nelumbo nucifera.* In one embodiment, leaves of the lotus plant are physically removed from the plant. The lotus leaves may then be inspected, cut and cleaned. In another embodiment, the compounds are isolated from dried leaves of the plant. In one embodiment, the lotus leaves are extracted by heating an aqueous solution of lotus leaves. Such an aqueous solution is heated to temperatures of about 60-100 degrees centigrade. The heated mixture may be filtered to remove the leaf debris. The filtered extract may be further spray dried to produce a fine powder. In one embodiment, the extract is prepared as such that 1g of extract powder is obtained from about 10 g of dried lotus leaf.

The lotus leaf may be from any of the variety of lotus plants, including, but not limited to, two similar genus in plant taxa: *Nelumbo* and *Nymphaea,* for example: *Nelumbo nucifera, Nelumbo lutea, Nymphaea alba, and Nymphaea caerulea.* The lotus leaf may also be from, but not limited to, any of lotus plants with the common names: Sacred Lotus, Blue Lotus, Yellow Lotus, White Lotus, European white water-lily, Egyptian blue lily, Orange Lotus, Golden Lotus, Red Lotus, Purple Lotus Pink Lotus and combinations thereof.

In one embodiment, the lotus leaf extract is *Nelumbo nucifera* extract.

The lotus leaf extract is typically combined with a cosmetically acceptable topical carrier into a topical composition.

The lotus leaf extract is used in a skin darkening effective amount. Such amount is often in the range of about 0.001 to about 20, preferably about 0.01 to about 10, more preferably about 0.1 to about 5, weight percent of the topical composition.

### Other Darkening Agents

In one embodiment, the topical composition further contains an additional darkening agent and/or a pigment, such as dihydroxyacetone, lawsone, erythulose, melanin, peptides, synthetic melanin derivatives, vanillin polymers, pigments, extracts such as but not limited to Coleus Forskoli extract, extracts from natural sources containing pigments (e.g., brown pigments from plants from the Hedychium genus or Bearberry genus or yellow, orange and red pigments from plants containing carotenoids or canthaxanthins); or synthetic chemicals such as compounds containing copper (e.g., copper salts such as CuCl₂) or synthetic carotenoids or canthaxantins. What is meant by an "extract" is a mixture of compounds isolated from a natural source (e.g., a plant).

Examples of synthetic melanin derivatives are disclosed in U.S. Patent Nos. 5,618,519, 5,384,116, and 5,227,459. Examples of soluble melanin derivatives are disclosed in 5,744,125, 5,225,435, 5,218,079, and 5,216,116. Examples of commercially available soluble melanin derivatives include Melasyn-100™ from San-mar laboratories, Inc. (Elmsford, NY) and MelanZe™ from Zylepsis (Ashford, Kent, United Kingdom).

These additional darkening agents will typically be present in the composition in an amount from about 0.001% to about 10% by weight.

In another embodiment, the composition may include a peptide. Examples of suitable peptides are described, e.g., in US Patent No. 7,081,442,"COMPOSITION CONTAINING A PEPTIDE AND A PIGMENT AND THE USE THEREOF IN DARKENING THE SKIN," US Patent No. 7,214,655 entitled "PEPTIDES AND THE USE THEREOF IN DARKENING THE SKIN," US Patent No. 6,797,697 entitled "COMPOSITION CONTAINING A PEPTIDE AND A PIGMENT AND THE USE THERE OF IN DARKENING THE SKIN," and U.S Patent No. 7,025,951 entitled "COMPOSITION AND METHODS FOR DARKENING THE SKIN."

### Topical Compositions

The compositions of the present invention are applied topically to human skin or hair. In one embodiment, the composition contains a skin darkening effective amount of a lotus leaf extract and a cosmetically acceptable topical carrier. In one embodiment, the cosmetically acceptable topical carrier is from about 50% to about 99.99%, by weight, of the composition (e.g., from about 80% to about 99%, by weight, of the composition). In a preferred embodiment of the invention, the cosmetically acceptable topical carrier includes or consists essentially of water.

The compositions may be made into a wide variety of product types that include but are not limited to lotions, creams, gels, sticks, sprays, ointments, cleansing liquid washes and solid bars, shampoos and hair conditioners, hair fixers, pastes, foams, powders, mousses, shaving creams, wipes, patches, hydrogels, film-forming products, facial masks and skin masks, films and make-up such as foundations, and mascaras. These product types may contain several types of cosmetically acceptable topical carriers including, but not limited to solutions, suspensions, emulsions such as microemulsions and nanoemulsions, gels, solids and liposomes. The following are non-limiting examples of such carriers. Other carriers can be formulated by those of ordinary skill in the art.

The topical compositions useful in the present invention can be formulated as solutions. Solutions typically include an aqueous or organic solvent (e.g., from about 50% to about 99.99% or from about 90% to about 99% of a cosmetically acceptable aqueous or organic solvent). Examples of suitable organic solvents include propylene glycol, polyethylene glycol (200-600), polypropylene glycol (425-2025), glycerol, 1,2,4-butanetriol, sorbitol esters, 1,2,6-hexanetriol, ethanol, and mixtures thereof.

Topical compositions useful in the subject invention may be formulated as a solution comprising an emollient. Such compositions preferably contain from about 2% to about 50% of an emollient(s). As used herein, "emollients" refer to materials used for the prevention or relief of dryness, as well as for the protection of the skin or hair. Examples of emollients include, but are not limited to, those set forth in the International Cosmetic Ingredient Dictionary and Handbook, eds. Pepe, Wenninger and McEwen, pp. 2930-36 (The Cosmetic, Toiletry, and Fragrance Assoc., Washington, D.C., 9th Edition, 2002) (hereinafter "ICI Handbook").

A lotion can be made from such a solution. Lotions typically contain from about 1% to about 20% (e.g., from about 5% to about 10%) of an emollient(s) and from about 50% to about 90% (e.g., from about 60% to about 80%) of water.

Another type of product that may be formulated from a solution is a cream. A cream typically contains from about 5% to about 50% (e.g., from about 10% to about 20%) of an emollient(s) and from about 45% to about 85% (e.g., from about 50% to about 75%) of water.

Although it is preferred that the composition of the present invention includes water, the composition may alternatively be anhydrous or an ointment that includes no water but organic and/or silicone solvents, oils, lipids and waxes. An ointment may contain a simple base of animal or vegetable oils or semi-solid hydrocarbons. An ointment may contain from about 2% to about 10% of an emollient(s) plus from about 0.1 % to about 2% of a thickening agent(s). Examples of thickening agents include, but are not limited to, those set forth in the ICI Handbook pp. 2979-84.

The composition may be formulated as an emulsion. If the topical carrier is an emulsion, from about 1% to about 10% (e.g., from about 2% to about 5%) of the topical carrier contains an emulsifier(s). Emulsifiers may be nonionic, anionic or cationic. Examples of emulsifiers include, but are not limited to, those set forth in the ICI Handbook, pp.2962-71.

Lotions and creams can be formulated as emulsions. Typically such lotions contain from 0.5% to about 5% of an emulsifier(s). Such creams typically contain from about 1% to about 20% (e.g., from about 5% to about 10%) of an emollient(s); from about 20% to about 80% (e.g., from 30% to about 70%) of water; and from about 1 % to about 10% (e.g., from about 2% to about 5%) of an emulsifier(s).

Single emulsion skin care preparations, such as lotions and creams, of the oil-in-water type and water-in-oil type are well-known in the cosmetic art and are useful in the subject invention. Multiphase emulsion compositions, such as the water-in-oil-in-water type or the oil-in-water-in-oil type, are also useful in the subject invention. In general, such single or multiphase emulsions contain water, emollients, and emulsifiers as essential ingredients.

The compositions of this invention can also be formulated as a gel (e.g., an aqueous, alcohol, alcohol/water, or oil gel using a suitable gelling agent(s)). Suitable gelling agents for aqueous and/or alcoholic gels include, but are not limited to, natural gums, acrylic acid and acrylate polymers and copolymers, and cellulose derivatives (e.g., hydroxymethyl cellulose and hydroxypropyl cellulose). Suitable gelling agents for oils (such as mineral oil) include, but are not limited to, hydrogenated butylene/ethylene/styrene copolymer and hydrogenated ethylene/propylene/styrene copolymer. Such gels typically contains between about 0.1 % and 5%, by weight, of such gelling agents.

The compositions of the present invention can also be formulated into a solid formulation (e.g., a wax-based stick, soap bar composition, powder, or a wipe containing powder).

The compositions useful in the subject invention may contain, in addition to the aforementioned components, a wide variety of additional oil-soluble materials and/or water-soluble materials conventionally used in compositions for use on skin and hair, at their art-established levels.

### Additional Cosmetically Active Agents

In one embodiment, the composition further contains another cosmetically active agent in addition to the lotus leaf extract or other darkening agent. As used herein, a "cosmetically active agent" is a compound (e.g., a synthetic compound or a compound isolated from a natural source or a natural extract) that has a cosmetic or therapeutic effect on the skin or hair, including, but not limiting to, anti-acne agents, shine control agents, anti-microbial agents, anti-inflammatory agents, anti-mycotic agents, anti-parasite agents, external analgesics, sunscreens, photoprotectors, antioxidants, keratolytic agents, detergents/surfactants, moisturizers, nutrients, vitamins, energy enhancers, anti-perspiration agents, astringents, deodorants, firming agents, anti-callous agents, and agents for hair and/or skin conditioning.

In one embodiment, the agent is selected from, but not limited to, the group consisting of hydroxy acids, benzoyl peroxide, D-panthenol, octyl methoxycinnimate, titanium dioxide, octyl salicylate, homosalate, avobenzone, carotenoids, free radical scavengers, spin traps, amines (e.g., DMAE and neutrol), retinoids such as retinol and retinyl palmitate, ceramides, polyunsaturated fatty acids, essential fatty acids, enzymes, enzyme inhibitors, minerals, hormones such as estrogens, steroids such as hydrocortisone, 2-dimethylaminoethanol, copper salts such as copper chloride, peptides containing copper such as Cu:Gly-His-Lys, coenzyme Q10, peptides, amino acids such as proline, vitamins, lactobionic acid, acetyl-coenzyme A, niacin, riboflavin, thiamin, ribose, electron transporters such as NADH and FADH2, and other botanical extracts such as aloe vera, feverfew, oatmeal and derivatives and mixtures thereof. The cosmetically active agent will typically be present in the composition of the invention in an amount of from about 0.001 % to about 20% by weight of the composition, e.g., about 0.005% to about 10% such as about 0.01 % to about 5%.

Examples of vitamins include, but are not limited to, vitamin A, vitamin Bs such as vitamin B3, vitamin B5, and vitamin B12, vitamin C, vitamin K, and different forms of vitamin E like alpha, beta, gamma or delta tocopherols or their mixtures, and derivatives thereof.

Examples of hydroxy acids include, but are not limited, to glycolic acid, lactic acid, malic acid, salicylic acid, citric acid, and tartaric acid.

Examples of antioxidants include, but are not limited to, water-soluble antioxidants such as sulfhydryl compounds and their derivatives (e.g., sodium metabisulfite and N-acetyl-cysteine), lipoic acid and dihydrolipoic acid, resveratrol, lactoferrin, and ascorbic acid and ascorbic acid derivatives (e.g., ascorbyl palmitate and ascorbyl polypeptide). Oil-soluble antioxidants suitable for use in the compositions of this invention include, but are not limited to, butylated hydroxytoluene, retinoids (e.g., retinol and retinyl palmitate), tocopherols (e.g., tocopherol acetate), tocotrienols, and ubiquinone. Natural extracts containing antioxidants suitable for use in the compositions of this invention, include, but not limited to, extracts containing flavonoids and isoflavonoids and their derivatives (e.g., genistein and diadzein), extracts containing resveratrol and the like. Examples of such natural extracts include grape seed, green tea, pine bark, and propolis.

### Other Materials

various other materials may also be present in the composition, as known in the art. These include humectants, pH adjusters, chelating agents (e.g., EDTA), minerals, and preservatives (e.g., parabens). Examples of such agents are listed in pp. 2922-23, 2926-28, and 2892 of the ICI Handbook. In addition, the compositions useful herein can contain conventional cosmetic adjuvants, such as dyes, opacifiers (e.g., titanium dioxide), and fragrances.

The composition and formulations and products containing such compositions of the present invention may be prepared using methodology that is well known by an artisan of ordinary skill.

The following non-limiting examples further illustrate the invention.

### Example 1: Lotus leaf extract increases melanin deposition in pigmented epidermal equivalents

Extracted powder of lotus leaf was purchased from Draco Natural Products, Inc. (San Jose, CA) and Jiangyin Tianjiang Pharmaceutical Co. Ltd (JiangYin, Jiangsu, P.R. China). Lotus seed powder was purchased from Jiangyin Tianjiang Pharmaceutical Co. Ltd (JiangYin, Jiangsu, P.R. China). Each powder was dissolved in water at a concentration of 10% (w/v) and boiled for 10 min to make stock solutions of lotus extracts. The stock solutions were stored at 4° C. Each stock solution (10%) was diluted with phosphate buffer saline (PBS) to the concentrations as indicated below.

The lotus extracts were tested for their ability to induce pigmentation in pigmented epidermal equivalents purchased from MatTek (Ashland, MA). The pigmented epidermal equivalents contain normal human melanocytes, together with normal, human-derived epidermal keratinocytes, which have been cultured to form a multi-layered, highly differentiated model of the human epidermis; and they were maintained according to manufacturer's instructions. The pigmented epidermal equivalents were treated topically with 15 □1 of PBS (vehicle control), or 0.1% or 0.5% lotus leaf extract, or 1% lotus seed extract, once daily, except weekends. At the 7^{th} day of the experiment, the pigmented epidermal equivalents were harvested for histological examination of pigment deposition using Fontana-Mason (F&M) staining (Sheenan DC, Hrapckak BB, eds: Theory and practice of Histo-Thchnology (St Louis: CV Mosby, 1980) pp 223-277). F&M staining identifies silver nitrate reducing activity, which, in skin, identifies melanin. Images of F&M stained equivalent sections were obtained and analyzed by ImagePro Plus 5.1 (Mediacybernetics, Silver Spring, MD). Melanin deposition (black pixels per surface area) was quantified using 12 images from each treatment in a single representative study. The results of such a study are presented in Table 1.

**Table 1**

| Treatment | Melanin deposition (average±SEM), normalized to vehicle control |
|---|---|
| PBS (vehicle control) | 100±12% |
| 0.1% lotus leaf extract (Draco Natural Products) | 205±16% |
| 0.5% lotus leaf extract | 130±9% |
| (Jiangyin Tianjiang Pharmaceutical Co. Ltd) | |
| 1% lotus seed extract | 107±9% |
| (Jiangyin Tianjiang Pharmaceutical Co. Ltd) | |

An increase in pigment deposition of 130-205% was seen in pigmented epidermal equivalents treated with lotus leaf extract, as compared to the PBS vehicle control. The darkening effect of lotus leaf extract was observed independently at least 3 times. In contrast, those equivalents treated with lotus seed extract showed only a 107 +/- 9 % increase in pigment deposition.

### Example 2: Lotus leaf extract increases pigment deposition in vivo

Lotus extracts (10% stock solutions of Example 1) were diluted with ethanol: propylene glycol, 70:30, v/v, to achieve a final solution of 5% lotus leaf or lotus seed extracts in 50% of water, 35% of ethanol and 15% of propylene glycol.

Yucatan swine (Sinclair Research Center, Columbia, MO) were housed in an appropriately sized cage in an environmentally controlled room with a 12h light/12 h dark photoperiod and supplied with food and water *ad libitum.* Animal care and use were according to the criteria described in the Guide for the Care and Use of Laboratory Animals, NIH publication 85-23. Swine were acclimated for a week before the start of treatments. Swine were topically treated with 5% lotus leaf extract, or 5% lotus seed extract (50µl, per 1 inch²), twice daily, five days per week, for 8 weeks. At the end of the treatment phase, skin biopsies were taken for F&M staining and histological analysis. Images of F&M stained skin sections were obtained by ImagePro Plus 5.1 (Mediacybernetics, Sliver Spring, MD). Melanin deposition (black pixels per surface area) was quantified by ImagePro Plus 5.1, using 12 images from each treatment. The untreated skins adjacent to the treated skin sites were used as controls to minimize possible variation of pigmentation in different locations of the swine skin. Table 2 represents the average melanin deposition of all sites of swine treated with each test material.

**Table 2**

| Treatment | Melanin level (average±SEM), normalized to control |
|---|---|
| Control | 100±7% |
| 5% lotus leaf extract | 184±12% |
| (Jiangyin Tianjiang Pharmaceutical Co. Ltd) | |
| 5% lotus seed extract | 126±13% |
| (Jiangyin Tianjiang Pharmaceutical Co. Ltd) | |

Histological analysis revealed an increase in pigment deposition in swine skin treated with lotus leaf extract (184+/-12%) and only a minimal increase in swine skin treated with lotus seed extract (126+/-13%).

It is understood that while the invention has been described in conjunction with the detailed description thereof, that the foregoing description is intended to illustrate and not limit the scope the invention, which is defined by the scope of the appended claims. Other aspects, advantages, and modifications are within the claims.

## Claims

1. A method of darkening the skin or hair comprising topically applying thereto a composition comprising a skin-darkening effective amount of a lotus leaf extract.

2. The method of claim 1, wherein said lotus leaf extract is selected from *Nelumbo nucifera, Nelumbo lutea, Nymphaea alba, Nymphaea caerulea,* Sacred Lotus, Blue Lotus, Yellow Lotus, White Lotus, European white water-lily, Egyptian blue lily, and combinations thereof.

3. The method of claim 1, wherein said lotus leaf extract is a *Nelumbo nucifera* leaf extract.

4. The method of any preceding claim, wherein said skin-darkening effective amount comprises about 0.001 to about 20 weight percent of the composition.

5. The method of any preceding claim, wherein said composition comprises a cosmetically acceptable topical carrier.

6. The method of claim 5, wherein said composition further comprises another darkening agent.

7. The method of any preceding claim, wherein said composition further comprises a pigment and/or dihydroxyacetone.

8. A composition for use in darkening the composition skin or hair, or evening skin tone, the composition comprising a skin-darkening effective amount of a lotus leaf extract, wherein the composition is topically applied to the skin or hair.

9. The composition of claim 8, wherein said lotus leaf extract is selected from *Nelumbo nucifera, Nelumbo lutea, Nymphaea alba, Nymphaea caerulea,* Sacred Lotus, Blue Lotus, Yellow Lotus, White Lotus, European white water-lily, Egyptian blue lily, and combinations thereof.

10. The composition of claim 8 or claim 9, wherein said lotus leaf extract is a *Nelumbo nucifera* leaf extract.

11. The composition of any one of claims 8 to 10 wherein said skin-darkening effective amount comprises about 0.001 to about 20 weight percent of the composition.

12. The composition of any one of claims 8 to 11, wherein said composition comprises a cosmetically acceptable topical carrier.

13. The composition of any one of claims 8 to 12, wherein said composition further comprises another darkening agent.

14. The composition of any one of claims 8 to 13, wherein said composition further comprises a pigment and/or dihydroxyacetone.

15. Use of a composition comprising a lotus leaf extract for darkening the skin or hair or evening the skin tone, wherein the composition is topically applied to the skin or hair.
